# FASCICULE DE BREVET EUROPEEN

(11) **EP 0 821 571 B1**
(45) Date de publication et mention de la délivrance du brevet: **30.09.1998**
(21) Numéro de dépôt: 96920869.3
(22) Date de dépôt: 30.05.1996
(51) Int. Cl.: A61B 17/50

(54) **INSTRUMENT DESTINE A RETIRER DES TIQUES PARASITES DE LA PEAU DES ANIMAUX OU DE L'HOMME**
INSTRUMENT ZUM RAUSZIEHEN VON ZECKEN AUS DER MENSCHLICHEN ODER TIERISCHEN HAUT
IMPLEMENT FOR REMOVING PARASITIC TICKS FROM THE SKIN OF ANIMALS OR HUMANS

(30) Priorité: 31.05.1995 FR 9506557
(43) Date de publication de la demande: 04.02.1998
(73) Titulaire: Heitz, Denis, 01590 Lavancia-Epercy (FR)
(72) Inventeur: Heitz, Denis, 01590 Lavancia-Epercy (FR)
(74) Mandataire: Myon, Gérard Jean-Pierre
(86) Numéro de dépôt international: FR9600812
(87) Numéro de publication internationale: WO9638095

(56) Documents cités:
- EP-A- 0 094 561
- FR-A- 520 169
- GB-A- 2 166 681
- US-A- 2 021 007
- US-A- 5 116 347
- US-A- 5 246 449

## Description

La présente invention concerne un instrument destiné à retirer des tiques parasites de la peau des animaux ou de l'homme, en particulier des animaux de compagnie et du bétail.

Les tiques, oui peuvent être à l'état de larves, de nymphes ou au stade adulte, se fixent dans la peau grâce à un rostre gui pénètre l'épiderme. Le retrait de ces tiques se fait traditionnellement avec de l'éther et des pinces de conception variée, mais il arrive souvent que la tête et le rostre de la tique restent plantés dans la peau.

Certaines pinces, telles que celle connue de US-A-5,246,449, permettent de retirer les tiques sans utilisation préalable d'éther, mais leur conception est plus complexe, et leur mode de préhension de la tique, en comprimant le corps de celle-ci, favorise la régurgitation de sang et de salive vers la peau de l'animal et donc la transmission de certaines maladies. De plus, la pince connue de US-A-5,246,449 agit par traction uniquement sur la tique, augmentant ainsi le risque de rupture du rostre dans la peau de l'hôte.

L'instrument, objet de cette invention, permet de remédier à ces inconvénients.

Dans cet esprit, l'invention concerne un instrument destiné à retirer des tiques parasites de la peau des animaux ou de l'homme, qui est constitué d'une seule pièce comportant, d'une part, un manche terminé par une prise de section ronde et, d'autre part, une extrémité recourbée et aplatie terminée par une fourche sensiblement perpendiculaire à l'axe de révolution de ladite prise, ladite fourche étant composée de deux dents délimitant un espace appelé entre-dent destiné à la préhension de ladite tique. Cet instrument est apte à retirer ladite tique par rotation autour d'un axe sensiblement perpendiculaire au plan de la peau sur laquelle la tique est fixée.

Grâce à l'invention, la tique peut être retirée de façon simple en insérant les dents de la fourche de part et d'autre du rostre et en faisant pivoter l'instrument autour d'un axe, de sorte que la tique n'est pas comprimée ni arrachée par simple traction mais au contraire retirée essentiellement par rotation.

Selon des modes particuliers de réalisation :
- Les deux dents peuvent avoir une section trapézoïdale de sorte que l'entre-dent a une forme de V en coupe verticale, permettant un meilleur maintien de la tique pendant l'extraction.
- Cet entre-dent peut avoir une largeur variable, en particulier avec une ouverture s'élargissant vers l'extrémité libre de la fourche, pour permettre à l'instrument de s'adapter à des tiques de tailles différentes.
- L'extrémité libre des deux dents peut être chanfreinée et arrondie.
- Les dents de la fourche peuvent être rectilignes ou présenter une courbure en direction du manche.
- La prise du manche peut présenter un diamètre supérieur à celui du manche.

L'invention sera mieux comprise et d'autres avantages de celle-ci apparaîtront plus clairement à la lumière de la description qui va suivre d'un mode de réalisation d'un instrument conforme à son principe, donnée uniquement à titre d'exemple et faite en référence aux dessins annexés dans lesquels :

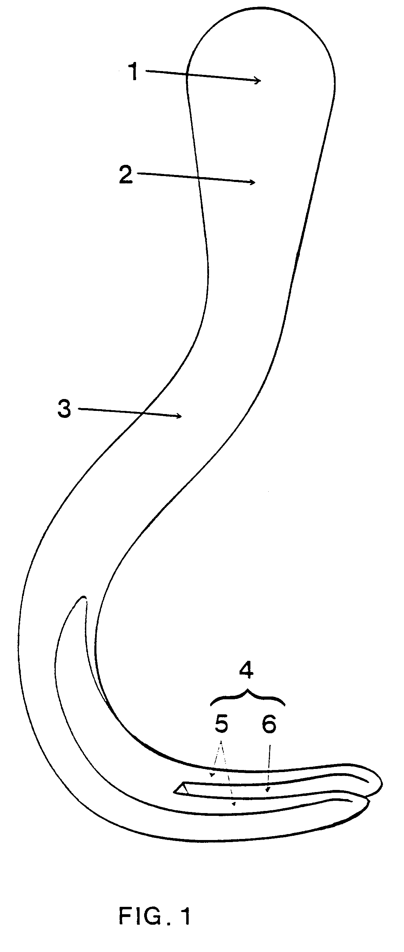

- La figure 1 est une vue d'ensemble de l'instrument ;

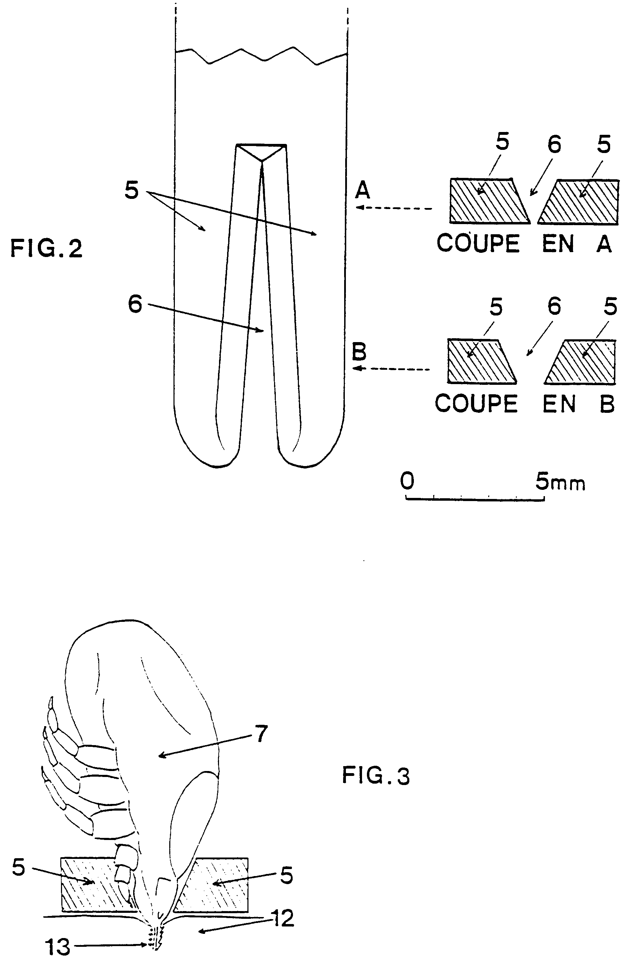

- La figure 2 est une vue du dessus de la fourche et comprend deux coupes transversales rapportées à cette vue ;
- La figure 3 montre le mode de préhension et de maintien de la tique et
- Les figures 4 et 5 présentent deux vues de profil de l'instrument engagé avec des tiques de tailles différentes.

Les dimensions données par l'échelle sont indiquées à titre d'exemple non limitatif.

En référence à ces dessins, l'instrument comporte :
- une tête hémisphérique 1 et une partie tronconique 2, l'ensemble constituant la prise de l'instrument,
- un manche en col de cygne 3 et
- une fourche 4 composée de deux dents 5 de section trapézoïdale, séparées par un espace appelé entre-dent 6.

L'entre-dent 6 présente une coupe transversale en forme de V vertical, pour venir s'adapter autour de la tête et du thorax de la tique 7, lorsque cette dernière est fixée dans la peau 12. Ainsi, l'abdomen de la tique n'est pas comprimé.

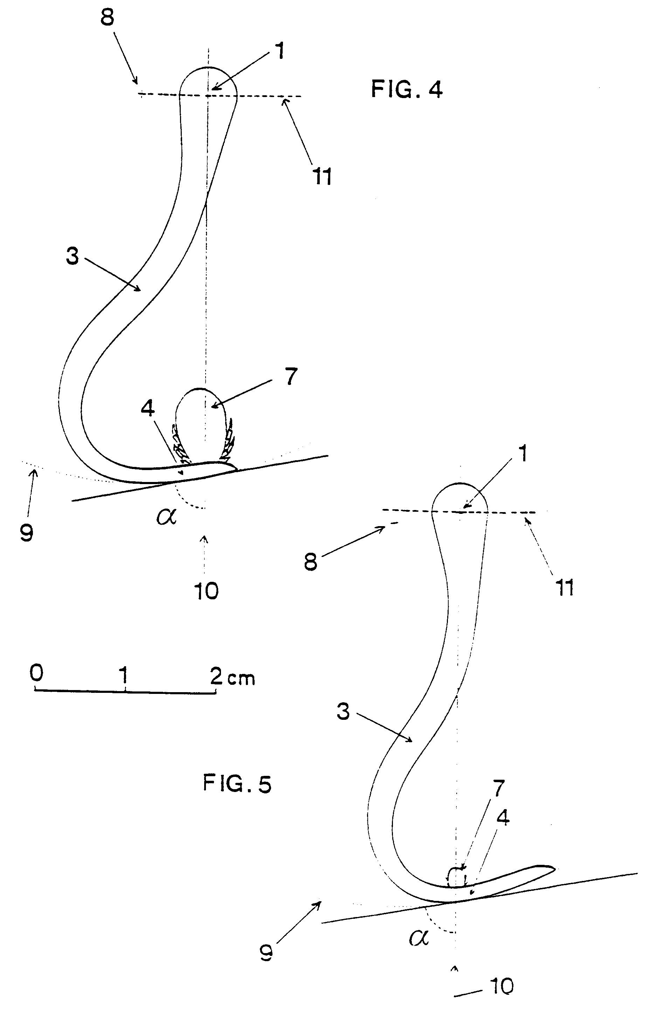

L'ouverture de l'entre-dent 6 s'élargit en allant vers l'extrémité, permettant à toutes les tiques, quelle que soit leur taille, de se trouver maintenues. En effet, comme il apparaît en comparant les figures 4 et 5, en fonction de la taille de la tique, la fourche est plus ou moins engagée de part et d'autre de la tique. Dans le cas d'une grosse tique, visible à la figure 4, celle-ci est peu enfoncée dans l'entre-dent 6 et les dimensions de l'entre-dent, au niveau de la coupe en B, permettent de ne pas comprimer la tique. Dans le cas d'une petite tique, visible à la figure 5, l'animal est profondément engagé dans l'entre-dent 6 et peut être fermement maintenu en place car l'entre-dent 6 est de dimensions relativement faibles comme il apparaît à la coupe en A.

A l'extrémité de la fourche 4, la face supérieure et les deux faces latérales de chaque dent 5 sont chanfreinées et les arêtes correspondantes arrondies pour faciliter le glissement de la fourche 4 de part et d'autre de la tique 7.

On définit l'axe de rotation 10 de l'instrument comme étant l'axe passant par le centre de la tête hémisphérique 1 et le rostre 13 de la tique 7. L'axe 10 est sensiblement perpendiculaire au plan de la peau sur laquelle la tique est fixée.

Le centre de la tête hémisphérique 1 est décalé en avant par rapport au centre 8 du cercle théorique 9 sur lequel s'incrivent les dents 5 de la fourche 4, de sorte que l'axe de rotation 10 forme toujours un angle aigü, α, compris entre 75 et 90° avec la fourche 4, quelle que soit la position de la tique 7 dans la fourche 4.

La forme de la tête 1 de l'instrument permet à l'opérateur de poser ses doigts dans le plan 11 perpendiculaire à l'axe de rotation 10, quelle que soit la position de la tique dans la fourche.

Le fonctionnement de l'instrument est le suivant :

L'opérateur tient l'instrument par la partie tronconique du manche. Il aborde la tique par un de ses côtés droit ou gauche, en posant la fourche sur la peau de l'animal ou de l'homme, les dents dirigées vers la tique. Il glisse la fourche sous la tique en engageant les dents de part et d'autre du corps du parasite, jusqu'à ce qu'elles parviennent à son contact étroit. Il exerce alors une très légère tension perpendiculaire à la peau, pour éviter que la tique n'échappe, et effectue une rotation, entre le pouce et l'index, de tout l'instrument. La tique se détache généralement au bout du deuxième ou du troisième tour. L'utilisation préalable d'éther ou d'autres produits acaricides est inutile.

La simplicité de conception de l'instrument permet d'envisager sa fabrication en matière plastique, en une seule étape d'injection, ou en métal moulé. L'instrument peut avantageusement être fabriqué en polyacétal qui présente la particularité de résister à l'alcool ou l'éther qui peuvent être utilisés pour nettoyer l'instrument après utilisation ou employés comme anesthésique par un utilisateur habitué a employer de tels produits.

## Revendications

1. Instrument destiné à retirer des tiques parasites de la peau des animaux ou de l'homme, comportant une extrémité recourbée et aplatie terminée par une fourche (4) composée de deux dents (5) délimitant un espace appelé entre-dents (6) destiné à la préhension de ladite tique (7), caractérisé en ce qu'il est constitué d'une seule pièce comportant, d'une part, un manche (3) terminé par une prise (1, 2) de section ronde et, d'autre part, ladite extrémité recourbée et aplatie, en ce que ladite fourche (4) est sensiblement perpendiculaire à l'axe de révolution de ladite prise et en ce que ledit instrument est apte à retirer ladite tique par une rotation autour d'un axe (10) sensiblement perpendiculaire au plan de la peau sur laquelle la tique est fixée.

2. Instrument, selon la revendication 1, caractérisé en ce que les deux dents (5) sont de section trapézoïdale, de sorte que ledit entre-dent (6) a une forme de V en coupe verticale.

3. Instrument, selon l'une des revendications 1 ou 2, caractérisé en ce que ledit entre-dent (6) a une largeur variable.

4. Instrument selon la revendication 3, caractérisé en ce que l'ouverture dudit entre-dent (6) s'élargit en allant vers l'extrémité libre de ladite fourche (4).

5. Instrument, selon l'une quelconque des revendications précédentes, caractérisé en ce que l'extrémité libre des dents (5) est chanfreinée et arrondie.

6. Instrument, selon l'une quelconque des revendications précédentes, caractérisé en ce que les dents (5) de la fourche (4) présentent une courbure en direction du manche.

7. Instrument, selon l'une quelconque des revendications précédentes, caractérisé en ce que la prise du manche (3) est une zone renflée constituée d'une partie tronconique (2) et d'une tête hémisphérique (1).

8. Instrument selon l'une quelconque des revendications précédentes, caractérisé en ce qu'il est réalisé en métal moulé.

9. Instrument selon l'une quelconque des revendications précédentes, caractérisé en ce qu'il est réalisé en matière plastique, tel qu'en polyacetal.

## Claims

1. Implement for removing parasitic ticks from the skin of animals or humans, including a curved and flattened end which finishes in a fork (4) made up of two tines (5) delimiting a since referred to as the tine interspace (6) which is intended for gripping the said tick (7); characterized in that it is made up of a single piece which includes, on the one hand, a handle (3) finishing in a grip (1, 2) of round cross-section, and, on the other hand, the said curved and flattened end; in that the said fork (4) is substantially perpendicular to the axis of revolution of the said grip; and in that the said implement is able to remove the said tick by rotation about an axis (10) substantially perpendicular to the plane of the skin on which the tick is fixed.

2. Implement according to Claim 1, characterized in that the two tines (5) are of trapezoidal cross-section, such that the said tine interspace (6) has a V shape in vertical section.

3. Implement according to one of Claims 1 and 2, characterized in that the said tine interspace (6) has a variable width.

4. Implement according to Claim 3, characterized in that the aperture of the said tine interspace (6) widens in the direction towards the free end of the said fork (4).

5. Implement according to any one of the preceding claims, characterized in that the free end of the tines (5) is bevelled and rounded.

6. Implement according to any one of the preceding claims, characterized in that the tines (5) of the fork (4) present a curvature in the direction of the handle.

7. Implement according to any one of the preceding claims, characterized in that the grip of the handle (3) is a bulging zone consisting of a frustoconical part (2) and off a hemispherical head (1).

8. Implement according to any one of the preceding claims, characterized in that it is made of cast metal.

9. Implement according to any one of the preceding claims, characterized in that it is made of plastic material, such as polyacetal.

## Patentansprüche

1. Instrument zum Ausziehen von parasitischen Zecken aus der Haut von Tieren oder Menschen mit einem gekrümmten und abgeflachten Endstück, das in einer Gabelform (4) endet, die aus zwei Zinken (5) besteht, zwischen denen ein Raum (6) zum Greifen der Zecke (7) gebildet ist, **dadurch gekennzeichnet,** daß das Instrument einstückig geformt, einerseits ein Aufnahmeteil (1,2) runden Querschnitts umfasst, das in einem Griff (3) endet und andererseits das gekrümmte und abgeflachte Endstück, wobei die Gabelform (4) genau rechtwinklig zur Achse des Aufnahmeteils verläuft und das Instrument die Zecke durch eine Drehung um eine Achse (10) auszieht, die rechtwinklig zur Ebene der Haut, in der die Zecke festsitzt, verläuft.

2. Instrument nach Anspruch 1, **dadurch gekennzeichnet,** daß die beiden Zinken (5) von trapezähnlichem Schnitt sind, so daß deren Zwischenraum (6) im Vertikalschnitt eine "V"-Form bildet.

3. Instrument nach einem der Ansprüche 1 oder 2, **dadurch gekennzeichnet,** daß der Zwischenraum (6) eine variable Breite aufweist,

4. Instrument nach Anspruch 3, **dadurch gekennzeichnet,** daß die Öffnung des Zwischenraums (6) sich zum freien Ende der Gabel (4) hin erweitert.

5. Instrument nach einem der vorhergehenden Ansprüche, **dadurch gekennzeichnet,** daß das freie Enden der Zinken (5) abgeschrägt und abgerundet ist

6. Instrument nach einem der vorhergehenden Ansprüche, **dadurch gekennzeichnet,** daß die Zinken (5) der Gabel (4) eine Krümmung in Richtung des Griffes aufweisen.

7. Instrument nach einem der vorhergehenden Ansprüche, **dadurch gekennzeichnet,** daß das Aufnahmeteil des Griffes (3) eine ausgebauchte Zone aufweist, die aus einem kegelstumpfartigen Teil (2) und einem halbkugelförmigen Kopf (1) besteht.

8. Instrument nach einem der vorhergehenden Ansprüche, **dadurch gekennzeichnet,** daß das Instrument aus Metall-Guss hergestellt ist.

9. Instrument nach einem der vorhergehenden Ansprüche, **dadurch gekennzeichnet,** daß Instrument aus Kunststoff, beispielsweise aus Polyazetal hergestellt ist.
